# EUROPEAN PATENT APPLICATION

(11) **EP 3 583 907 A1**
(43) Date of publication of application: **25.12.2019**
(21) Application number: 18178515.5
(22) Date of filing: 19.06.2018
(51) Int. Cl.: A61B 18/20, A61B 18/00

(54) **RESTRICTING OSCILLATION AND COLLAPSE OF CAVITATION BUBBLES**

(71) Applicant: Koninklijke Philips N.V., 5656 AE Eindhoven (NL)
(72) Inventor: Thumma, Kiran Kumar, 5656 AE Eindhoven (NL); Baragona, Marco, 5656 AE Eindhoven (NL); Jurna, Martin, 5656 AE Eindhoven (NL); Boamfa, Marius Iosif, 5656 AE Eindhoven (NL)
(74) Representative: de Haan, Poul Erik

(57) **Abstract**

The invention discloses an apparatus for use in a hair cutting device (100), the hair cutting device having an optical waveguide (102) having a sidewall, wherein a portion of the sidewall forms a cutting face for contacting a hair on the body of a subject, the apparatus comprising a disruption element configured to restrict the oscillation and collapse of a cavitation bubble at the optical waveguide.

## Description

### FIELD OF THE INVENTION

The invention relates to an apparatus for use with a hair cutting device for cutting (e.g. shaving) hair on the body of the subject and, in particular, to an apparatus capable of restricting the oscillation and collapse of cavitation bubbles. The invention also relates to a cutting assembly and the hair cutting device including such an apparatus.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular, a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

When light from the fiber optic couples into a hair to be cut, or debris of hair that has already been cut, the temperature of the fiber optic may increase rapidly. If fluid (e.g. water or shaving gel) is in contact with the fiber optic during such a rapid temperature increase, a vapor cavity may form that may act as a cavitation source (also referred to as a cavitation bubble). Cavitation is the term used to describe the formation (and collapse) of the vapor cavities (i.e. liquid-free regions, such as "bubbles" or "voids") in a liquid in a region of very high temperature and low pressure. Liquids go through different boiling regimes at different temperatures. An optical waveguide/fiber optic can reach temperatures of around 600 or 700 degrees centigrade, and such a high temperature may cause liquid in contact with the optical waveguide to undergo a number of different boiling regimes. Such regimes include nucleate boiling and film boiling (and the transition between the two), which will be familiar to those skilled in the art. The temperature gradient in the liquid moving away from the fiber optic is very steep, and this steep temperature gradient causes cavitation bubbles formed at the fiber optic to move to a region of lower temperature and high pressure, away from the fiber optic. When the bubble is in a region of relatively high pressure (and/or density), the bubble/void implodes or collapses under the pressure of the surrounding liquid. During cavitation, shock waves may be formed, particularly during the oscillatory formation and collapse of a bubble and, if the bubble collapses on, or near to the fiber optic, then the generated shock waves may cause significant damage to the fiber optic. In addition, the liquid-vapor phase change at the surface of the bubble/cavity may cause and/or contribute to the pressure shock wave.

Therefore, it would be desirable to have a cutting device having an optical waveguide cutting arrangement with a reduced likelihood of damage occurring as a result of oscillating and collapsing cavitation bubbles.

### SUMMARY OF THE INVENTION

When cutting hair (or contacting hair debris), the temperature of the cutting element of the cutting device increases significantly, and reaches temperatures at which cavitation occurs in a liquid. In order to reduce the likelihood of the cutting element becoming damaged as a result of shock waves formed by the oscillation and/or collapse of a cavitation bubble, it would be desirable to reduce the number of significant oscillation/collapse events occurring at the cutting device. According to embodiments of the present disclosure, this may be achieved by restricting the number of such events that can occur at the cutting device. For example, in a first embodiment, the volume or depth of liquid within which the cutting device is submerged is limited so that significant cavitation bubbles cannot be formed. In a second embodiment, cavitation bubbles that might oscillate and collapse are drawn away from the cutting device so that any shock waves generated by the collapse do not damage the cutting device.

According to a first aspect, an apparatus for use in a hair cutting device is disclosed. The hair cutting device has an optical waveguide having a sidewall, wherein a portion of the sidewall forms a cutting face for contacting a hair on the body of a subject. The apparatus comprises a disruption element configured to restrict the oscillation and collapse of a cavitation bubble at the optical waveguide.

The disruption element may function in one of a number of ways to prevent, or restrict cavitation bubbles from oscillating and collapsing at, or close to, the optical waveguide. In this way, shock waves that may result from the collapse of a cavitation bubble may have little or no effect on the optical waveguide and, therefore, damage to the optical waveguide may be reduced or prevented. This, in turn, may help to increase the life of the optical waveguide and the hair cutting device to which it is attached.

In some embodiments, the disruption element may be configured to limit, to a defined level, a depth of fluid on the body in which a cavitation bubble may form. The disruption element may, in some embodiments, comprise a blade to displace a portion of fluid from the body of the subject.

In some embodiments, the disruption element may be configured to limit the depth of fluid on the body to between around 50 micrometers and 100 micrometers.

The disruption element may extend for a length substantially equal to or greater than a length of the cutting face of the optical waveguide.

In some embodiments, the disruption element may be positioned ahead of the cutting face of the optical waveguide in the direction of motion during use.

The disruption element may comprise a surface spaced apart from the optical waveguide so as to attract a cavitation bubble away from the optical waveguide, towards the surface.

The surface may be configured to engage with a cavitation bubble forming in the fluid, so as to cause the cavitation bubble to collapse.

In some embodiments, the surface of the disruption element may be spaced apart from the optical waveguide by a distance of between around 500 micrometers and 1000 micrometers.

The disruption element may, in some embodiments, be moveable relative to the optical waveguide.

According to a second aspect, a cutting assembly for use in a hair cutting device comprises an optical waveguide having a sidewall, wherein a portion of the sidewall forms a cutting face for contacting a hair on the body of a subject. The cutting assembly also comprises an apparatus as disclosed herein.

The optical waveguide may comprise an optical fiber.

According to a third aspect, a hair cutting device for cutting hair on a body of the subject comprises a light source for generating laser light at one or more specific wavelengths corresponding to wavelengths absorbed by one or more chromophores in hair; an optical waveguide coupled to the light source to receive laser light, the optical waveguide having a sidewall, wherein a portion of the sidewall forms a cutting face for contacting a hair on the body of a subject; and an apparatus as disclosed herein.

According to a fourth aspect, a hair cutting device for cutting hair on the body of a subject comprises a light source for generating laser light at one or more specific wavelengths corresponding to wavelengths absorbed by one or more chromophores in hair; and a cutting assembly having a cutting element coupled to the light source to receive laser light, the cutting assembly comprising a cutting assembly as disclosed herein.

The hair cutting device may further comprise a body engagement element for engaging a portion of the body of the subject as the hair cutting device is moved over the body. The apparatus may be mounted to the body engagement element.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified schematic of a hair cutting device according to embodiments;
Fig. 2 is a pair of schematic drawings showing different views of an exemplary hair cutting device according to embodiments;
Fig. 3 is a graph illustrating the refractive index of hair;
Fig. 4 is a simplified schematic of an example of an apparatus according to embodiments;
Fig. 5 is a simplified schematic of a further example of an apparatus according to embodiments;
Fig. 6 is a simplified schematic of a further example of an apparatus according to embodiments;
Fig. 7 is a simplified schematic of a further example of an apparatus according to embodiments;
Fig. 8 is a simplified schematic of an example of a cutting assembly according to embodiments;
Fig. 9 is a simplified schematic of an example of a hair cutting device according to embodiments; and
Fig. 10 is a simplified schematic of a further example of a hair cutting device according to embodiments.

### DETAILED DESCRIPTION OF THE EMBODIMENTS

As noted above, the present invention provides an improvement in the cutting ability and efficiency of a laser light-based shaving device, for example as described in WO 2014/143670. In particular, it has been recognized that restricting the oscillation and collapse of cavitation bubbles that the optical waveguide (e.g. at the cutting face of the optical waveguide) can help to reduce damage or failure of the optical waveguide by shock waves resulting from the cavitation events.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

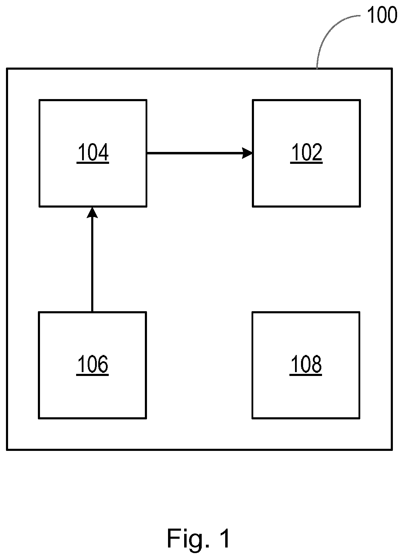

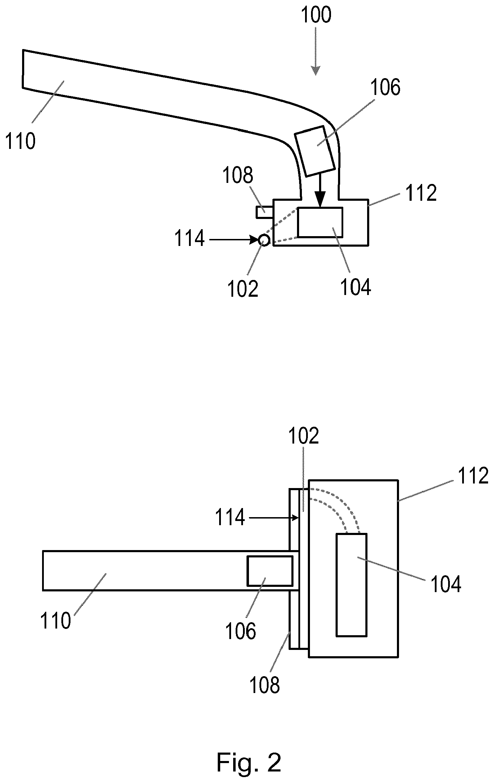

Fig. 1 is a block diagram of a hair cutting device 100 according to an embodiment of the invention. Fig. 2 shows a hair cutting device 100 in the form of a handheld razor according to an exemplary embodiment of the invention. The hair cutting device 100 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 100 comprises a cutting element 102 that enables hair to be cut as the hair cutting device 100 is moved over the skin of a subject. The cutting element 102 is an optical waveguide 102 that is arranged on the hair cutting device 100 so that the optical axis of the optical waveguide 102 (i.e. the line along which light typically propagates through the optical waveguide 102) is generally perpendicular to the direction in which the hair cutting device 100 is moved so that hairs contact the sidewall of the optical waveguide 102 (the sidewall corresponding to at least a portion of the long edge of the optical waveguide 102) as the hair cutting device 100 is moved across the skin of the subject. In some embodiments, the optical waveguide 102 is an optical fiber, although those skilled in the art will be aware of other types of optical waveguide that can be used according to the invention, such as a slab waveguide, a strip waveguide or a photonic crystal waveguide. An optical fiber comprises a core, and in some embodiments also comprises a cladding, which may or may not fully encompass the core (e.g. part of the core may be exposed).

A light source 104 is provided in the hair cutting device 100 that generates laser light at one or more specific wavelengths. The light source 104 is optically coupled to the optical waveguide 102. The laser light generated by the light source 104 is coupled into the optical waveguide 102 (and specifically coupled into an end of the optical waveguide so that the laser light propagates through the optical waveguide).

The light source 104 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its color. Thus, the laser light will be absorbed by the chromophore and converted into heat which will melt or burn the hair or otherwise destroy the bonds in the molecules of the hair, and it is this melting or burning that provides the cutting action of the hair cutting device 100.

Suitable chromophores that can be targeted by the laser light generated by the light source 104 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 104 should generate for this purpose, and further details are not provided herein.

In some embodiments the light source 104 can be configured to generate laser light at a plurality of wavelengths (either simultaneously or sequentially), with each wavelength being selected to target a different type of chromophore. This can improve the cutting action of the optical waveguide 102 since multiple types of molecules in the hair may be burnt using the laser light. Alternatively multiple light sources 104 can be provided that each generate laser light at a respective wavelength, and each light source 104 can be coupled to a respective optical waveguide 102 to provide multiple cutting elements 102 in the device 100.

The hair cutting device 100 may also comprise a control unit 106 to control the operation of the hair cutting device 100. The control unit 106 may be connected to the light source 104 to control the activation and deactivation of the light source 104 (and in some embodiments control the wavelength and/or intensity of the light generated by the light source 104). The control unit 106 may activate and deactivate the light source 104 in response to an input from a user of the hair cutting device 100. The control unit 106 can comprise one or more processors, processing units, multi-core processors or modules that are configured or programmed to control the hair cutting device 100. In some embodiments, the control unit may receive instructions from other controllers or processors remote from the device 100.

The hair cutting device 100 may also comprise an apparatus 108 for restricting the oscillation and collapse of a cavitation bubble at the optical waveguide 102, as discussed in detail below.

As noted above, Fig. 2 shows a hair cutting device 100 that is in the form of a handheld wet razor. Fig. 2 shows a side view and a bottom view of the razor 100. The razor 100 comprises a handle 110 for the subject (or other user of the device 100) to hold, and a head portion 112 that includes the cutting element 102 (optical waveguide/fiber). As shown, the optical waveguide 102 is arranged along an edge of the head portion, and a part of the optical waveguide 102 forms (or corresponds to) a cutting face 114. The cutting face 114 is the part of the optical waveguide 102 that is intended to come into contact with hair as the hair cutting device 100 is moved across the skin of the subject. The light source 104 and the control unit 106 are shown as being incorporated into the head portion 112 and handle 110 respectively, but it will be appreciated that the positions of these components in the hair cutting device 100 as shown in Fig. 2 is not limiting. Likewise it will be appreciated that the embodiment shown in Fig. 2 is merely an example, and the invention can be incorporated or used in any type of hair cutting device 100 that comprises an optical waveguide cutting element 102 as described herein. The apparatus 108 is shown to be mounted above the optical waveguide 102, but it will be appreciated that this is only one example of the position of the apparatus 108, and other arrangements are possible, as discussed below. As noted above, the optical waveguide 102 may form part of a cutting assembly which may itself form a part of, or be detachably connected to, the head portion 112 and/or to the handle 110.

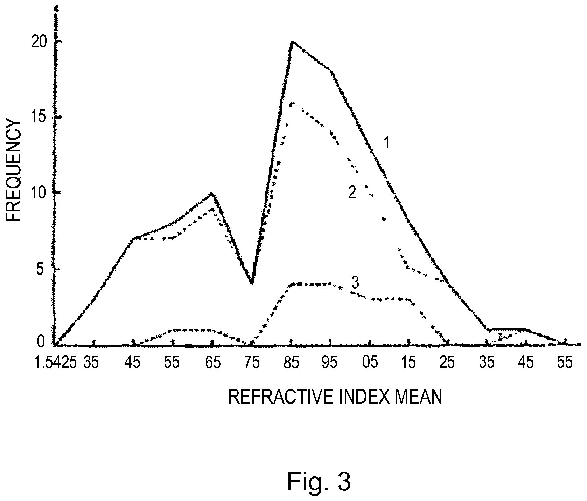

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognizes that the refractive index of hair can depend on the sex of the subject, e.g. the refractive index of hair on a female is generally higher than the refractive index of hair on a male.

As is known, the optical waveguide 102 acts as a waveguide for the light coupled from the light source 104 through the occurrence of total internal reflection, since the refractive index of air is lower than that of the optical waveguide 102. However, if an object that has a refractive index higher than the optical waveguide 102 is put into contact with the optical waveguide 102, then the total internal reflection is 'frustrated' and light can couple from the optical waveguide 102 into that object. Thus, in order for light to be coupled into a hair from the optical waveguide 102 (to provide the cutting action according to the invention), the optical waveguide 102 should generally have the same or a lower refractive index than hair at the point at which the hair contacts the optical waveguide 102. Thus, the optical waveguide 102 should have the same or a lower refractive index than hair at least at the cutting face 114 portion of the optical waveguide 102. Preferably the refractive index of the optical waveguide 102 at the cutting face 114 is the same as that of hair since that provides the best coupling of light from the optical waveguide 102 to the hair. However, in cases where the refractive index of the optical waveguide 102 is higher than an object (e.g. a hair) in contact with the optical waveguide, less efficient output coupling can still be achieved by increasing the numerical aperture (NA) and, preferably, using the full NA. One technique which may be used to reach the full NA is to introduce local tapering in the optical waveguide at or near to the cutting region.

Thus, in some embodiments, the refractive index of the optical waveguide 102 at least at the cutting face 114 is equal to or lower than 1.56. More preferably the refractive index of the optical waveguide 102 at least at the cutting face 114 is equal to or lower than 1.55. Even more preferably, the refractive index of the optical waveguide 102 at least at the cutting face 114 is equal to or lower than 1.54, since this refractive index is below the refractive indices identified in Fig. 3.

In some embodiments, a lower bound for the refractive index of the optical waveguide 102 at the cutting face 114 can be 1.48, 1.51, 1.53 or 1.54.

A range of values from which the refractive index of the optical waveguide 102 is selected can be formed from any combination of the upper and lower refractive index bounds set out in the preceding paragraphs.

The optical waveguide/fiber 102 can be made from any suitable material or combination of materials. For example optical waveguides/fibers can be composed of or comprise silica, fluoride glass, phosphate glass, chalcogenide glass, crown glass (such as BK7) and/or crystals (such as sapphire or yttrium aluminum garnet (YAG)).

As discussed above, the cutting device 100 may be used in a wet shave environment, for example using water or a lubricant, such as oil, foam or gel to form a lubricating layer on the body of the subject (e.g. on the skin) to be shaved. Applying such a fluid to the surface of the skin prior to shaving or cutting may also help to improve the hair cutting efficiency and/or quality. When at least a portion of the cutting face 114 of the optical waveguide 102 is in contact with the liquid, then cavitation bubbles may form if that portion of the optical waveguide increases significantly in temperature (e.g. if it comes into contact with a hair or any portion of hair). When such cavitation bubbles expand and collapse, particularly in a rapid, oscillatory manner, shock waves, or pressure waves, may be formed. The strength of the shock wave may depend on (amongst other factors) the size of the cavitation bubble formed and the speed at which the cavitation bubble collapses. If the shock wave is strong enough, and is close to the optical waveguide 102, then the optical waveguide may be damaged. However, if the layer of liquid on the body of the subject is thin enough, then the size of a cavitation bubble formed in the liquid will be restricted. Thus, some embodiments disclosed herein relate to a mechanism by which the volume or depth of liquid on the body of the subject is limited or restricted. Other embodiments disclosed herein relate to a mechanism by which cavitation bubbles that are formed are drawn away from the optical waveguide 102 such that, when the bubbles collapse, they do so at a safe distance from the optical waveguide to avoid or at least mitigate any significant adverse effect on the optical waveguide.

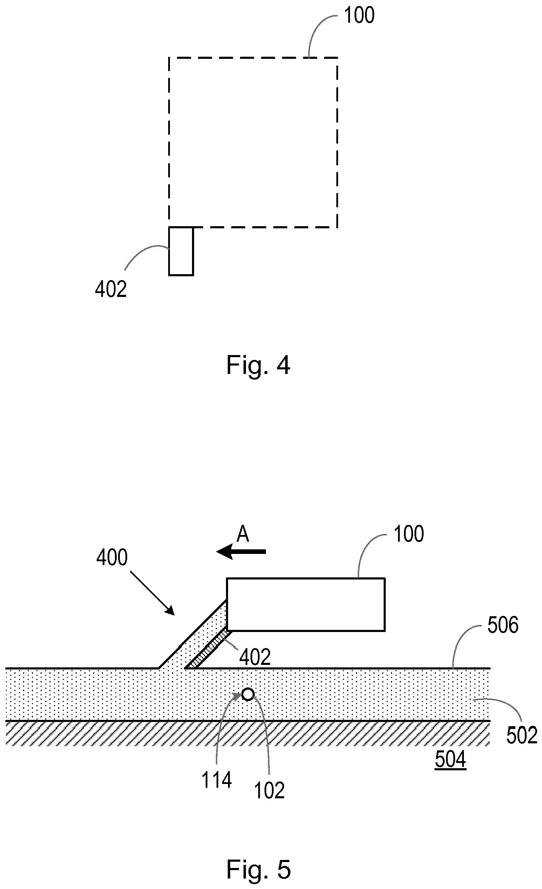

Fig. 4 is a simplified schematic of an example of an apparatus 400 for use in a hair cutting device. Such a hair cutting device (shown in Fig. 4 having reference 100, and shown in dashed lines to indicate that it is not essential part of the apparatus 400) has an optical waveguide (e.g. the optical waveguide 102) having a sidewall, wherein a portion of the sidewall forms a cutting face (e.g. the cutting face 114) for contacting a hair on the body of a subject. The apparatus 400 comprises a disruption element 402 to restrict the oscillation and collapse of a cavitation bubble at the optical waveguide 102. The apparatus 400 may comprise the apparatus 108 discussed above. In some embodiments, the disruption element 402 may restrict the oscillation and collapse of a cavitation bubble in the vicinity of the optical waveguide 102. Here, a cavitation bubble may be considered to be "in the vicinity of' the optical waveguide 102 if it is close enough that, upon collapsing, it would cause damage to the optical waveguide. As will become apparent from the discussion below, the disruption element 402 may take on various forms, and may function in different ways, depending on the manner in which it is used to restrict the oscillation and collapse of cavitation bubbles. While, in Fig. 4, the disruption element 402 is shown in a particular position relative to the cutting device 100, it will be appreciated that, according to various embodiments, the disruption element 402 may be positioned and/or oriented differently.

Fig. 5 is a simplified schematic of one example of the apparatus 400. Other features of the cutting device 100 have been omitted from Fig. 5 for clarity. In this embodiment, the disruption element 402 serves as a blade or scraping element. Fig. 5 shows a layer of fluid 502 (e.g. water, shaving gel, shaving foam or shaving oil) formed over at least part of a body 504 (e.g. skin) of a subject. During the shaving or cutting activity, the optical waveguide 102 is positioned at least partially within the layer of fluid 502 to enhance the hair cutting efficiency. During the cutting activity, the cutting device 100 may be moved over the body 504 of the subject in a direction indicated by the arrow A. In some embodiments, the direction of movement of the cutting device 100 may be such that the cutting face 114 of the optical waveguide 102 is at the leading side of the optical waveguide, so as to engage with hairs coming into contact with the optical waveguide. As the cutting device 100 is moved over the body 504 of the subject in the direction A, the disruption element 402 moves or displaces a portion of the fluid 502 from the body of the subject, so as to reduce the depth of the layer of fluid on the body. The disruption element 402 may be configured (e.g. sized and shaped) such that fluid 502 from the layer of fluid is scooped or scraped out of the path of the cutting face 114 of the optical waveguide 102. For example, fluid 502 may be moved to one or both sides of the optical waveguide 102 by the disruption element 402. Thus, in some embodiments, the disruption element 402 may have a shape similar to a plough (e.g. a snow plough). While, in the example of Fig. 5, the disruption element 402 is shown (schematically) to be substantially flat, in other examples, the disruption element 402 may be curved. In some embodiments, the disruption element 402 may extend for a length substantially equal to, or greater than, a length of the cutting face 114 of the optical waveguide 102. In this way, the entire length of the cutting face 114 of the optical waveguide 102 can be protected from damage caused by the oscillation and collapse of cavitation bubbles.

The disruption element 402 may be arranged such that a defined depth of fluid 502 remains on the body 504 for the optical waveguide 102 to pass through. The depth of the remaining fluid 502 may be defined such that there is insufficient fluid for a large cavitation bubble to form at the optical waveguide 102. In some embodiments, the depth of the remaining fluid 502 may be limited to approximately the size of cavitation bubbles growing during the nucleate boiling phase, i.e. around 50 micrometers (µm) to 100 µm. If a cavitation bubble were to form in the fluid 502 remaining in the layer of fluid, then it would reach an air/fluid interface 506 before the cavitation bubble grows large enough to collapse in a potentially-damaging way or to enter into a bubble forming/collapsing oscillating pattern. When such a cavitation bubble reaches the air/fluid interface 506, the gas contained within the cavitation bubble would be vented out into the air.

Thus, in some embodiments, such as the embodiment shown in Fig. 5, the disruption element 402 may be configured to limit, to a defined level, a depth of fluid on the body in which a cavitation bubble may form. As shown in Fig. 5, the disruption element 402 may, in some embodiments, comprise a blade to displace a portion of fluid from the body of the subject. In some embodiments, the disruption element 402 may be configured to limit the depth of fluid on the body to between around 50 µm to around 100 µm. In other embodiments, the disruption element 402 may be configured to limit the depth of the on the body to a different defined level, for example to between around 20 µm and around 500 µm between around 25 µm and around 200 µm, between around 50 µm and around 150 µm or between around 75 µm and around 100 µm.

In some embodiments, the disruption element 402 may be positioned ahead of the cutting face 114 of the optical waveguide 102 in the direction of motion during use. For example, in the embodiment shown in Fig. 5, the cutting device 100 moves in the direction A during use, and the disruption element 402 is positioned ahead of (e.g. downstream of) the optical waveguide 102. The disruption element 402 may, in some embodiments, be positioned only slightly ahead of the optical waveguide 102 in the direction of motion during use, so that the disruption element does not adversely affect the position and/or orientation of any hairs that it contacts prior to contact being made with the hairs by the optical waveguide. For example, the disruption element 402 may engage the top of a hair, but the optical waveguide 102 should still be able to make contact with the hair at a different point, depending on the desired hair cutting length.

While the optical waveguide 102 of the embodiment shown in Fig. 1 has a single cutting face 114, in other embodiments, the optical waveguide may include multiple cutting faces 114 (e.g. a cutting face on opposing sides of the optical waveguide. In such embodiments, the disruption element 402 may be located and/or oriented differently, or multiple disruption elements may be provided. For example, the first disruption element 402 may be configured to displace liquid on the body of the subject when the cutting device 100 is moved in a first direction, and a second disruption element may be configured to displace liquid on the body of the subject when the cutting device is moved in a second direction.

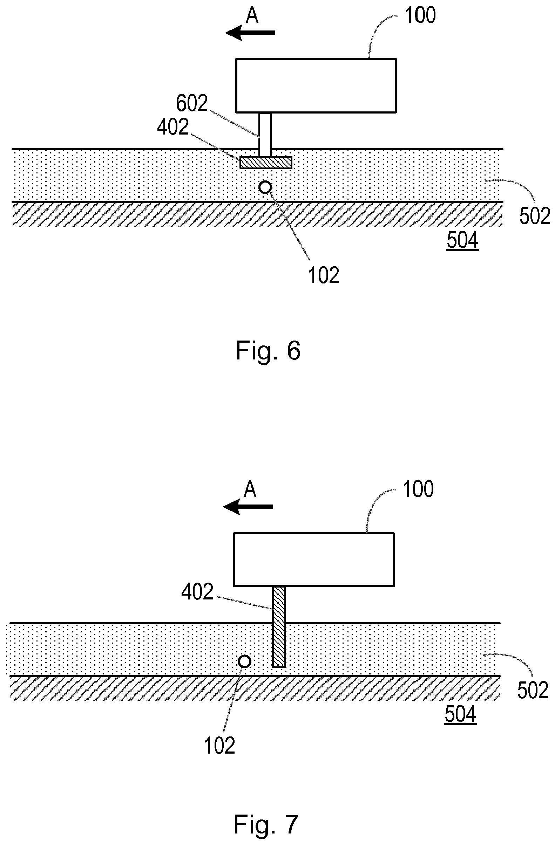

Figs. 6 and 7 show alternative examples of the apparatus 400 according to various embodiments. In the embodiments shown in Figs. 6 and 7, the disruption element 402 is not configured to displace liquid 502 from the body 504 of the subject. Rather, in these embodiments, the disruption element 402 is intended to draw cavitation bubbles away from the optical waveguide 102, so that, when a shock wave is formed as a result of the oscillatory formation and collapse of a cavitation bubble, the cavitation bubble is far enough away from the optical waveguide to prevent the optical waveguide from being damaged by the shock wave.

It has been found that, when a cavitation bubble is formed near to (e.g. in the vicinity of) a rigid surface, then the cavitation bubble tends to collapse towards the rigid surface, and the shock wave generated as a result of the collapse is directed towards the rigid surface. Thus, by positioning a surface, such as a rigid surface (e.g. the disruption element 402), in a position spaced apart from the optical waveguide, but close enough to draw any cavitation bubbles away from the optical waveguide 102 through fluid motion, the cavitation bubbles will be drawn/moved towards the rigid surface (e.g. the disruption element 402) so that the shock wave formed by the collapse of the cavitation bubble does not damage the optical waveguide 102. Thus, according to some embodiments (such as the embodiments shown in Figs. 6 and 7), the disruption element 402 may comprise a surface spaced apart from the optical waveguide 102 so as to attract a cavitation bubble away from the optical waveguide, towards the surface. The disruption element 402 may, therefore, comprise a rigid element. The surface (e.g. the disruption element 402) may be configured to engage with a cavitation bubble forming in the fluid, so as to cause the cavitation bubble to collapse.

In the example of Fig. 6, the disruption element 402 is connected to the cutting device 100 by a mounting element 602. The disruption element 402 is mounted above the optical waveguide 102, such that any cavitation bubbles formed at the optical waveguide are drawn towards the disruption element, away from the optical waveguide and the body 504 of the subject.

In Fig. 7, the disruption element 402 is connected to the cutting device directly. The disruption element 402 may alternatively be connected to the cutting device using any known mounting means. In this embodiment, the disruption element 402 is mounted behind the optical waveguide 102 in the direction of motion of the optical waveguide during use such that cavitation bubbles that are formed at the optical waveguide 102 are drawn towards the disruption element 402, behind the optical waveguide. In this way, the disruption element 402 is positioned such that it will not engage with a hair to be cut, prior to the hair cutting into contact with the optical waveguide 102.

As noted above, the surface (e.g. the disruption element 402) is positioned away from the optical waveguide 102 such that cavitation bubbles are drawn a sufficient distance away from the optical waveguide to prevent damage to the optical waveguide when cavitation bubbles collapse, but close enough to the optical waveguide that the attraction is strong enough to draw the bubbles away from the optical waveguide. Thus, in some embodiments, the surface of the disruption element 402 may be spaced apart from the optical waveguide 102 by a distance approximately equal to the maximum radius of a cavitation bubble. Thus, in some embodiments, the surface of the disruption element 402 may be spaced apart from the optical waveguide 102 by a distance of between around 200 µm to around 5000 µm. In other embodiments, the distance between the surface of the disruption element 402 and the optical waveguide 102 may be different, for example between around 300 µm and around 3000 µm, or between around 400 µm and 2000 µm, or between around 500 µm and around 1000 µm.

The disruption element 402 may, in some embodiments, be movable relative to the optical waveguide 102. Thus, whether the disruption element 402 is configured to displace liquid 502 from the body 504 of the subject, as in the embodiment of Fig. 5, or configured to attract cavitation bubbles away from the optical waveguide, as in the embodiments of Figs. 6 and 7, the disruption element 402 may be movably mounted to enable it to move relative to the optical waveguide. For example, the disruption element 402 may be mounted in such a way as to allow it to move the between its position shown in Fig. 6 and its position shown in Fig. 7. Similarly, the disruption element 402 shown in Fig. 5 may be movable between a first side of the optical waveguide and a second side of the optical waveguide. Enabling the position of the disruption element 402 to be moved may allow for the cutting device 100 to be used in a bidirectional manner, for example with a cutting face 114 located either side of the optical waveguide 102.

Although not shown in the drawings, the apparatus 400 may, in some embodiments, comprise multiple disruption elements 402. For example, the apparatus 400 may comprise a first disruption element 402 for displacing liquid 502 (as shown in the embodiment of Fig. 5) and a second disruption element for attracting cavitation bubbles away from the optical waveguide 102 (as shown in the embodiments of Figs. 6 and 7). This may further reduce the risk of damage being caused to the optical waveguide 102 by the installation and collapse of cavitation bubbles.

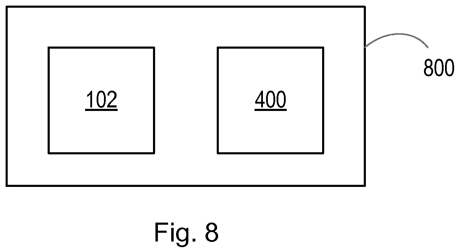

So far, embodiments have been described in terms of the apparatus 400 which may be connected to, coupled to or otherwise mounted to the cutting device 100. In some embodiments, the apparatus 400 may form part of an assembly which can be detachably attached to a cutting device. Thus, according to a further aspect, a cutting assembly is provided. Fig. 8 is a simplified schematic of an example of a cutting assembly 800 for use of the hair cutting device, such as the hair cutting device 100. The cutting assembly 800 comprises an optical waveguide 102 having a sidewall, wherein a portion of the sidewall forms a cutting face 114 for contacting a hair on the body 504 of a subject. The cutting assembly 800 further comprises an apparatus 400 as disclosed herein. The cutting assembly 800 may, for example, comprise a disposable attachment which can be removed and disposed of if, for example, any part of the cutting assembly (e.g. the optical waveguide 102) becomes worn or damaged. The worn or damaged cutting assembly 800 may then be replaced with a new cutting assembly. In this way, if any part of the cutting assembly 800 is worn or damaged, the user is able to replace only a part of the cutting device 100, rather than the whole cutting device.

In any of the embodiments disclosed herein, the optical waveguide 102 may comprise an optical fiber. In alternative embodiments, the optical waveguide may comprise some other type of waveguide.

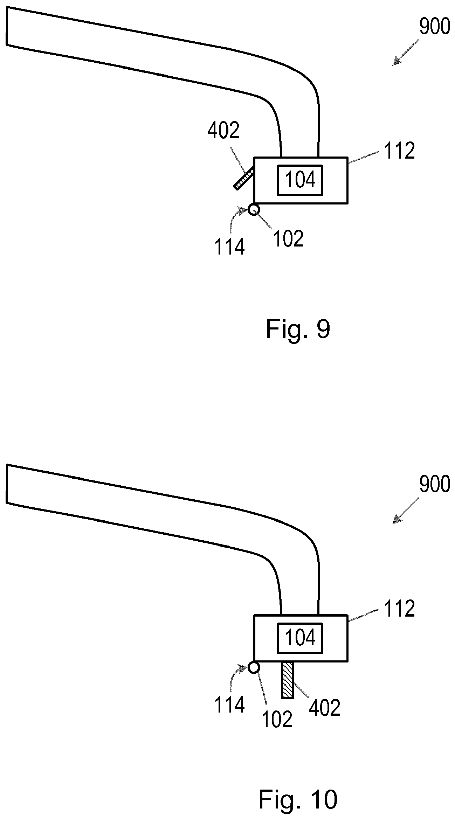

According to some embodiments, the apparatus 400 may form part of a hair cutting device, such as the hair cutting device 100. Thus, according to a further aspect, a hair cutting device is provided. Figs. 9 and 10 are simplified schematics of an example of a hair cutting device 900 for cutting hair on a body of a subject. The hair cutting device 900 comprises a light source 104 for generating laser light at one or more specific wavelengths corresponding to wavelengths absorbed by one or more chromophores in hair; an optical waveguide 102 coupled to the light source to receive laser light, the optical waveguide having a sidewall, wherein a portion of the sidewall forms a cutting face 114 for contacting a hair on the body of a subject; and an apparatus 400 as disclosed herein.

Fig. 9 shows an example of a hair cutting device 900 having a disruption element 402 similar to that shown in Fig. 5. In this embodiment, the disruption element 402 is configured to scrape or displace liquid 502 from the body 504 of a subject, so as to reduce or restrict the depth of liquid to a defined level, so that large cavitation bubbles are unable to form. Fig. 10 shows an example of a hair cutting device 900 having a disruption element 402 similar to that shown in Figs. 6 and 7. In this embodiment, the disruption element 402 comprises a surface spaced apart from the optical waveguide 102, is configured to attract cavitation bubbles away from the optical waveguide, towards the surface.

According to a further aspect, a further hair cutting device is provided. Thus, a hair cutting device 900 cutting hair on a body of a subject may comprise a light source 104 for generating laser light at one or more specific wavelengths corresponding to wavelengths absorbed by one or more chromophores in hair; and a cutting assembly 800 having a cutting element 102 coupled to the light source to receive laser light. Thus, the cutting assembly may comprise a cutting assembly 800 as disclosed herein. The cutting element may comprise the optical waveguide 102 as disclosed herein. As discussed above, the cutting assembly 800 may be removable from (e.g. detachably connected to) a handle portion of the hair cutting device 900.

According to some embodiments, the hair cutting device 900 may further comprise a body engagement element (not shown) for engaging a portion of the body 504 of the subject as the hair cutting device is moved over the body. The apparatus 400 may be mounted to the body engagement element. A body engagement element may be used to apply a force to the body 504 of the subject on which the cutting device 900 is being used. The body engagement element may stretch the skin during a hair cutting activity, to draw the hair out of the skin, so as to achieve a closer shave or a cleaner cut. Such a body engagement element may be positioned ahead of the optical waveguide 102 along the path of motion during use. Due to the position of such a body engagement element relative to the optical waveguide 102, the body engagement element may provide a convenient structure on which to mount the apparatus 400.

Thus, embodiments disclosed herein provide an effective way of restricting the oscillation and collapse of cavitation bubbles at (or in the vicinity of) the optical waveguide of a cutting device, thereby reducing the likelihood that shock waves generated by the collapse of the cavitation bubble will damage the optical waveguide. Thus, the life of an optical waveguide in such a cutting device may be lengthened.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An apparatus (400) for use in a hair cutting device, the hair cutting device having an optical waveguide having a sidewall, wherein a portion of the sidewall forms a cutting face (114) for contacting a hair on the body of a subject, the apparatus comprising:
a disruption element (402) configured to restrict the oscillation and collapse of a cavitation bubble at the optical waveguide.

2. An apparatus (400) according to claim 1, wherein the disruption element (402) is configured to limit, to a defined level, a depth of fluid on the body in which a cavitation bubble may form.

3. An apparatus (400) according to claim 1 or claim 2, wherein the disruption element (402) comprises a blade to displace a portion of fluid from the body (504) of the subject.

4. An apparatus (400) according to claim 2 or claim 3, wherein the disruption element (402) is configured to limit the depth of fluid on the body (504) to between around 50 micrometers and 100 micrometers.

5. An apparatus (400) according to any of the preceding claims, wherein the disruption element (402) extends for a length substantially equal to or greater than a length of the cutting face of the optical waveguide (102).

6. An apparatus (400) according to any of the preceding claims, wherein the disruption element (402) is positioned ahead of the cutting face of the optical waveguide (102) in the direction of motion during use.

7. An apparatus (400) according to claim 1, wherein the disruption element (402) comprises a surface spaced apart from the optical waveguide (102) so as to draw a cavitation bubble away from the optical waveguide, towards the surface.

8. An apparatus (400) according to claim 7, wherein the surface is configured to engage with a cavitation bubble forming in the fluid, so as to cause the cavitation bubble to collapse.

9. An apparatus (400) according to claim 7 or claim 8, wherein the surface of the disruption element (402) is spaced apart from the optical waveguide (102) by a distance of between around 500 micrometers and 1000 micrometers.

10. An apparatus (400) according to any of the preceding claims, wherein the disruption element (402) is moveable relative to the optical waveguide (102).

11. A cutting assembly (800) for use in a hair cutting device, the cutting assembly comprising:
an optical waveguide (102) having a sidewall, wherein a portion of the sidewall forms a cutting face (114) for contacting a hair on the body (504) of a subject; and
an apparatus (400) according to any of the preceding claims.

12. A cutting assembly (800) according to claim 11, wherein the optical waveguide (102) comprises an optical fiber.

13. A hair cutting device (900) for cutting hair on a body (504) of a subject, the hair cutting device comprising:
a light source (104) for generating laser light at one or more specific wavelengths corresponding to wavelengths absorbed by one or more chromophores in hair;
an optical waveguide (102) coupled to the light source to receive laser light, the optical waveguide having a sidewall, wherein a portion of the sidewall forms a cutting face (114) for contacting a hair on the body of a subject; and
an apparatus (400) according to any of claims 1 to 10.

14. A hair cutting device (900) for cutting hair on a body of a subject, the hair cutting device comprising:
a light source (104) for generating laser light at one or more specific wavelengths corresponding to wavelengths absorbed by one or more chromophores in hair; and
a cutting assembly (800) having a cutting element (102) coupled to the light source to receive laser light, the cutting assembly comprising a cutting assembly according to claim 11 or claim 12.

15. A hair cutting device (900) according to claim 13 or claim 14, further comprising:
a body engagement element for engaging a portion of the body of the subject as the hair cutting device is moved over the body;
wherein the apparatus (400) is mounted to the body engagement element.
